# EUROPEAN PATENT SPECIFICATION

(11) **EP 3 118 330 B1**
(45) Date of publication and mention of the grant of the patent: **11.09.2019**
(21) Application number: 16179730.3
(22) Date of filing: 15.07.2016
(51) Int. Cl.: C12Q 1/682, C12Q 1/6825, C12Q 1/6804, C12Q 1/6832, G01N 33/552, C07H 21/04, G01N 33/53

(54) **DETECTION COMPRISING SIGNAL AMPLIFIER**
DETEKTION MIT EINEM SIGNALVERSTÄRKER
AMPLIFICATEUR COMPRENANT UN SIGNAL DE DÉTECTION

(30) Priority: 15.07.2015 US 201562192987 P
(43) Date of publication of application: 18.01.2017
(73) Proprietor: Helios Bioelectronics Inc., Hsinchu County 30261 (TW)
(72) Inventor: Wai-Hong, Chan, Hardy, Redwood City, CA 94065 (US); Yuh-Shyong, Yang, 300 Hsinchu (TW); Wen-Yih, Chen, 320 Taoyuan City (TW)
(74) Representative: Fuchs Patentanwälte Partnerschaft mbB

(56) References cited:
- US-A1- 2007 231 790
- ANRAN GAO ET AL: "Enhanced Sensing of Nucleic Acids with Silicon Nanowire Field Effect Transistor Biosensors", NANO LETTERS, vol. 12, no. 10, 10 October 2012 (2012-10-10), pages 5262-5268, XP055325402, US ISSN: 1530-6984, DOI: 10.1021/nl302476h
- YASUHIDE OHNO ET AL: "Label-Free Biosensors Based on Aptamer-Modified Graphene Field-Effect Transistors", JOURNAL OF THE AMERICAN CHEMICAL SOCIETY, vol. 132, no. 51, 29 December 2010 (2010-12-29), pages 18012-18013, XP055100068, ISSN: 0002-7863, DOI: 10.1021/ja108127r

## Description

### FIELD OF THE INVENTION

This invention relates to a molecular detection technique. More particularly, the invention relates to a detection comprising a signal amplifier.

### BACKGROUND OF THE INVENTION

Molecular detection plays an important role in clinical diagnosis and molecular biology research. Several systems have been developed to perform molecular detection for detecting and/or identifying a target molecule in a sample.

A field-effect transistor (FET) is a semiconductor electronic component extremely sensitive to a surface charge. Among several kinds of FET, silicon nanowire (SiNW) FET biosensor refers to a sensing element where the surface of silicon nanowires is modified with a recognizing molecule. When the FET is exposed to an aqueous environment comprising a target molecule such as proteins, DNA, or RNA, the target molecule binds to the recognizing molecule on the surface of the silicon nanowire field-effect transistor. In this case, the electric field formed by the electrical charges carried by the target molecule affects the number of electrons or holes of the SiNW-FET, triggering a rise or fall of electrical conductivity. By monitoring the change of the electrical conductivity, the presence and even the concentration of the target molecule can be determined.

The field-effect transistor has been employed to detect and/or identify a target oligonucleotide as well as a protein-protein binding, both of which benefit from the absence of labeling requirements for reagents and the ready availability of commercial manufacturing sources for FET sensors. Sensitivity of the FET sensor is highly dependent on detection distance (debye length) between the transistor surface and the actual detected molecules. Most current types of FET are less than satisfactory as gene detection devices in terms of sensitivity. This is mainly due to the requirement of relatively high salt concentrations for DNA/DNA or DNA/RNA hybridizations. Hybridization of highly charged biomolecules requires an appropriate ionic strength to suppress the charge repulsive forces. Unfortunately, ions in the hybridization buffer also reduce the FET debye length and hence diminish detection sensitivity. In the case of antibody-antigen binding detection, the large size of the antibody as compared to other biomolecules also reduces the detection sensitivity of the FET. For example, in the detection applying a secondary antibody, the net charges of the secondary antibody in a sensing zone (within the debye length) are less in view of the large size of the secondary antibody, and the change of the electrical conductivity is less; thus the signal is weak. The surface charge distribution of the antibody and the binding orientation of the bound antibody make FET detection difficult to be resolved and quantitatively analyzed. In addition, medium concentrations of salt in the binding buffer also reduce the debye length and, in turn, lower the detection sensitivity as well.

### SUMMARY OF THE INVENTION

In order to improve detection sensitivity and sensing limit, a detection method comprising a signal amplifier is provided.

The invention is to provide a method for detecting a target molecule, comprising forming a capturing complex comprising the target molecule; and binding the capturing complex with a signal amplifier, wherein the capturing complex has a net electrical charge; the signal amplifier has affinity to the capturing complex and has alike net electrical charge of the net electrical charge of the capturing complex, wherein the method further comprises forming the capturing complex with a recognizing molecule which is able to capture the target molecule with affinity, wherein the recognizing molecule comprises a partially neutral single-stranded oligonucleotide comprising at least one electrically neutral nucleotide and at least one negatively charged nucleotide, wherein the method further comprises monitoring an electrical change occurring due to the binding of the capturing complex and the signal amplifier, and wherein the capturing complex is attached on a solid surface or the capturing complex is spaced apart from the solid surface by a distance of about 0 to about 10 nm.

The present invention is also to provide a kit for detecting a target molecule, comprising:
a signal amplifier having affinity to a capturing complex comprising the target molecule, wherein the capturing complex has a net electrical charge; the signal amplifier has a like net electrical charge of the net electrical charge of the capturing complex; and
an electrical signal detecting element for detecting the electrical change occurring due to the binding of the capturing complex and the signal amplifier;
a recognizing molecule which is able to capture the target molecule with affinity to form the capturing complex, wherein the recognizing molecule comprises a partially neutral single-stranded oligonucleotide comprising at least one electrically neutral nucleotide and at least one negatively charged nucleotide; and
a solid surface, wherein the capturing complex is attached on the solid surface or the capturing complex is spaced apart from the solid surface by a distance of about 0 to about 10 nm.

The present invention is described in detail in the following sections. Other characteristics, purposes and advantages of the present invention can be found in the detailed description and claims.

### BRIEF DESCRIPTION OF THE DRAWINGS

FIG. 1 shows a schematic drawing of a method.
FIG. 2 shows one preferred embodiment of the electrically neutral nucleotide according to the invention.
FIG. 3 shows I_{D}-V_{G} curve of the detection using R18 RNA aptamer as a signal amplifier for amplifying a primary signal of 6X histidine tag antigen and anti-6X histidine-tag rabbit antibody.
FIG. 4 shows I_{D}-V_{G} curve of the detection using anti-rabbit goat antibody as a signal amplifier for amplifying a primary signal of 6X histidine tag antigen and anti-6X histidine-tag rabbit antibody.
FIG. 5 shows the threshold voltage shift induced by the detection using R18 RNA aptamer or anti-rabbit goat antibody as a signal amplifier to amplify a primary signal of 6X histidine tag antigen and anti-6X histidine-tag rabbit antibody.

### DETAILED DESCRIPTION OF THE INVENTION

The invention is to provide a method for detecting a target molecule, comprising forming a capturing complex comprising the target molecule; and binding the capturing complex with a signal amplifier, wherein the capturing complex has a net electrical charge; the signal amplifier has affinity to the capturing complex and has alike net electrical charge of the net electrical charge of the capturing complex, wherein the method further comprises forming the capturing complex with a recognizing molecule which is able to capture the target molecule with affinity, wherein the recognizing molecule comprises a partially neutral single-stranded oligonucleotide comprising at least one electrically neutral nucleotide and at least one negatively charged nucleotide, wherein the method further comprises monitoring an electrical change occurring due to the binding of the capturing complex and the signal amplifier, and wherein the capturing complex is attached on a solid surface or the capturing complex is spaced apart from the solid surface by a distance of about 0 to about 10 nm.

As used herein, the term "detecting" refers to discovering or determining the existence or presence of a target molecule, and preferably, identifying the target molecule. In one preferred embodiment of the invention, detecting comprises quantifying the target molecule in a sample. A reaction applied herein includes but is not limited to hybridization between oligonucleotide molecules.

As used herein, the term "a target molecule" refers to a specified macromolecule to be detected or identified from a pool of molecules. The target molecule is a macromolecule such as a nucleotide, oligonucleotide or polynucleotide. The target molecule is naturally occurring or artificial. In another aspect, the target molecule is purified or mixed with other contents. In one preferred embodiment of the invention, the expression pattern of the target molecule is different in a normal condition and in an abnormal condition, such as a disease. In another preferred embodiment of the invention, the expression pattern of the target molecule is different in different cell types. In yet another preferred embodiment of the invention, the target molecules are DNA molecules or RNA molecules. The DNA molecules are preferably a gene or an untranscripted region. The RNA molecules are preferably mRNA, micro RNA, long untranslated RNA, rRNA, tRNA, or siRNA.

The sample according to the invention is derived from a naturally occurring origin or derived from artificial manipulation. Preferably, the sample is derived from a naturally occurring origin such as an extract, body fluid, tissue biopsy, liquid biopsy, or cell culture. In another aspect, the sample is processed according to the reaction required for detection. For example, the pH value or ion strength of the sample may be adjusted.

As used herein, the term "a capturing complex" refers to a complex comprising at least two molecules and one of the molecules in the capturing complex is the target molecule. One of other molecules in the capturing complex is able to specifically bind to the target molecule, i.e. to capture the target molecule with affinity, for forming the capturing complex. The method according to the invention comprises forming the capturing complex with a recognizing molecule which is able to capture the target molecule with affinity. The forming of the capturing complex provides a primary signal for discovering or determining the existence or presence of the target molecule. The type of the recognizing molecule depends on the type of the target molecule.

In one preferred embodiment of the invention, the recognizing molecule is a single-stranded oligonucleotide molecule able to form the capturing complex with a target oligonucleotide molecule according to base complementarity. The capturing complex preferably refers to a double-stranded structure, and one single strand is the target oligonucleotide molecule and the other single strand is the recognizing molecule. Preferably, the recognizing molecule has a sequence matched to the sequence of the target oligonucleotide molecule; more preferably, has a sequence perfectly matched to the sequence of the target oligonucleotide molecule. By forming the capturing complex, the target oligonucleotide molecule can be captured from a mixture in the sample. The capturing step also refers to a purification step of specifically selecting the target oligonucleotide molecule and presenting the target oligonucleotide molecule in the capturing complex.

The target oligonucleotide molecule can be a single-stranded molecule or a double-stranded molecule. The manner of obtaining the single strand of the double-stranded target oligonucleotide molecule can be, for example, heating or changing ion strength of the environment of the double-stranded target oligonucleotide molecule.

The manner for forming the capturing complex depends on the natural properties of the target molecule and the recognizing molecule. Examples of the manner for forming the capturing complex include, but are not limited to, hybridization between oligonucleotide molecules.

The capturing complex is attached on a solid surface or the capturing complex is spaced apart from the solid surface by a distance of about 0 to about 10 nm.

As used herein, the term "solid surface" refers to a solid support including but not limited to a polymer, paper, fabric, or glass. The solid surface to be employed varies depending on the signal to be detected. For example, when the method adopts a field-effect transistor to monitor the signal, the solid surface is a transistor surface of the field-effect transistor; when the method adopts a surface plasmon resonance, the solid surface is a metal surface of a surface plasmon resonance.

In a preferred embodiment of the invention, the material of the solid surface is silicon; preferably polycrystalline silicon or single crystalline silicon; more preferably polycrystalline silicon. Polycrystalline silicon is cheaper than single crystalline silicon, but because the polycrystalline has more grain boundary, a defect usually occurs in the grain boundary that hinders electron transduction. Such phenomenon makes the solid surface uneven and quantification difficult. Furthermore, ions may penetrate into the grain boundary of the polycrystalline and cause detection failure in solution. In addition, polycrystalline silicon is not stable in air. The abovementioned drawbacks, however, would not interfere with the function of the method according to the invention.

The manner of attaching the capturing complex on the solid surface depends on the material of the solid surface and the type of capturing complex. In one embodiment of the invention, the capturing complex links to the solid surface through a covalent bond. Examples of the covalent bond include but are not limited to the following methods, depending on the solid surface chemistry and the target molecule or the recognizing molecule. In one embodiment of the invention, when silicon oxide is used as the solid surface, the solid surface is modified by using (3-Aminopropyl)triethoxysilane (APTES). The silicon atom in the molecule of APTES performs a covalent bond with the oxygen atom of the hydroxyl group and it converts the surface's silanol groups (SiOH) to amines; then the 5'-amino group of recognizing single-stranded oligonucleotide molecule is covalently bonded with the solid surface amines group by glutaraldehyde (Roey Elnathan, Moria Kwiat, Alexander Pevzner, Yoni Engel, Larisa Burstein, Artium Khatchtourints, Amir Lichtenstein, Raisa Kantaev, and Fernando Patolsky, Biorecognition Layer Engineering: Overcoming Screening Limitations of Nanowire-Based FET Devices, Nano letters, 2012, 12, 5245-5254). In another embodiment of the invention, the solid surface is modified into self-assembling monolayer molecules with different functional groups for covalently linking to different functional groups of the recognizing single-stranded oligonucleotide molecule by various chemical reactions (Srivatsa Venkatasubbarao, Microarrays - status and prospects, TRENDS in Biotechnology Vol. 22 No. 12 December 2004; Ki Su Kim, Hyun-Seung Lee, Jeong-A Yang, Moon-Ho Jo and Sei Kwang Hahn, The fabrication, characterization and application of aptamer-functionalized Si-nanowire FET biosensors, Nanotechnology 20 (2009)).

In another preferred embodiment of the invention, the capturing complex is spaced apart from the solid surface by a distance. Since an electrical change detecting element is applied for detecting the electrical change due to the binding of the capturing complex and the signal amplifier, the capturing complex is not necessary to directly bind to the solid surface, provided that the distance between the capturing complex and the solid surface is short enough to allow the electrical change detecting element to detect the electrical change. The distance between the solid surface and the capturing complex is about 0 to about 10 nm; preferably about 0 to about 5 nm.

The capturing complex according to the invention has a net electrical charge. Preferably, an electrical signal produced by the net electrical charge of the capturing complex is too weak to be detected, and a signal amplifier is needed.

As used herein, the term "a signal amplifier" refers to a molecule that has affinity to the capturing complex, and has alike net electrical charge of the net electrical charge of the capturing complex. If the net electrical charge of the capturing complex is positive, the like net electrical charge of the signal amplifier is also positive; if the net electrical charge of the capturing complex is negative, the like net electrical charge of the signal amplifier is also negative. Although the signal amplifier has the like net electrical charge of the net electrical charge of the capturing complex, the affinity binding between the signal amplifier and capture complex is stronger than the repelling force between these two net electrical charges. By binding the signal amplifier and the capturing complex, the signal of the net electrical charge of the capturing complex is amplified by introducing the like net electrical charge of the signal amplifier.

Preferably, the signal amplifier has a small size. With the small size, the signal amplifier and the capture complex bound are located in a sensing zone of an electrical signal detecting element, and the amplifying effect is maximized. For example, when applying FET as the electrical signal detecting element, the signal amplifier and the capture complex bound are located within the debye length. More preferably, the molecular weight of the signal amplifier is from about 0.5 kDa to about 50 kDa; still more preferably from about 1.5 kDa to about 35 kDa.

In another aspect, the signal amplifier has abundant net electrical charges. Preferably, the signal amplifier has at least one net electrical charge, more preferably, at least five net electrical charges; still more preferably, at least ten electrical charges.

In one preferred embodiment of the invention, the signal amplifier has high net charge density. A molecule having high net charge density means that the molecule has more net charges compared to a molecule with the same molecular weight. More preferably, the signal amplifier has abundant net electrical charges and a small size.

The signal amplifier has affinity to the target molecule or recognizing molecule in the capture complex. Preferably, the signal amplifier has affinity to the target molecule.

In one preferred embodiment of the invention, the signal amplifier is an oligonucleotide molecule, including a single-stranded oligonucleotide molecule and a double-stranded oligonucleotide molecule. By having different sequences and/or structures, an oligonucleotide is able to have affinity to different molecules. In one more preferred embodiment of the invention, the signal amplifier is an oligonucleotide aptamer.

As used herein, the term "an oligonucleotide" or "an oligonucleotide molecule" refers to an oligomer of nucleotide. The term "nucleotide" refers to an organic molecule composed of a nitrogenous base, a sugar, and one or more phosphate groups; preferably one phosphate group. The nitrogenous base includes a derivative of purine or pyrimidine. The purine includes substituted or unsubstituted adenine and substituted or unsubstituted guanine; the pyrimidine includes substituted or unsubstituted thymine, substituted or unsubstituted cytosine and substituted or unsubstituted uracil. The sugar is preferably a five-carbon sugar, more preferably substituted or unsubstituted ribose or substituted or unsubstituted deoxyribose. The phosphate groups form bonds with the 2, 3, or 5-carbon of the sugar; preferably, with the 5-carbon site. For forming the oligonucleotide, the sugar of one nucleotide is joined to the adjacent sugar by a phosphodiester bridge. Preferably, the oligonucleotide is DNA or RNA; more preferably DNA.

As used herein, the term "an oligonucleotide aptamer" refers to an oligonucleotide molecule that binds to a specific molecule. The oligonucleotide aptamer can be obtained by selecting it from a large random sequence pool, such as from the SELEX (systematic evolution of ligands by exponential enrichment) method. In responding to the capture complex, a qualified oligonucleotide aptamer can be selected.

According to the invention, if the target molecule is present in the sample, the recognizing molecule binds to the target molecule to form the capturing complex with the net electrical charge. Because the capturing complex carries the net electrical charge, an electrical change as a primary signal occurs due to the capturing complex forming by introducing the net electrical charges. On the other hand, if the target molecule is absent from the sample, the recognizing molecule fails to bind to the target molecule. Therefore, the electrical environment is unchanged, and no electrical change occurs. Consequently, if the capturing complex is present in the sample, the signal amplifier binds to the capturing complex. Because the signal amplifier carries the like net electrical charge of the net electrical charge of the capturing complex, an amplified electrical change occurs due to the signal amplifier binding by introducing more net electrical charges. Monitoring the electrical change occurring, the target molecule is detected thereby. Thus, the method according to the invention further comprises monitoring an electrical change occurring due to the binding of the capturing complex and the signal amplifier.

The electrical change according to the invention includes but is not limited to increase of the net electrical charges. The electrical change can be detected as an electrical signal. The electrical signal includes but is not limited to changes of electric conductivity, electric field, electric capacitance, electric current, electron, or electron hole. In one preferred embodiment of the invention, the electrical change is a threshold voltage shift change. Preferably, the electric change is detected by an electric change detecting element.

Preferably, the solid surface is coupled with the electrical change detecting element for detecting the electrical change. Preferably, the electrical change detecting element is a field-effect transistor or a surface plasmon resonance, more preferably, field-effect transistor. Examples of field-effect transistor include but not limited to nanowire field-effect transistor, nanotube field-effect transistor and graphene field-effect transistor.

According to the invention, a free form of the signal amplifier is preferably removed before detecting the electrical change, thereby avoiding noise generated by the electrical charges carried by the free form of the signal amplifier. The manner of removal includes by is not limited to washing.

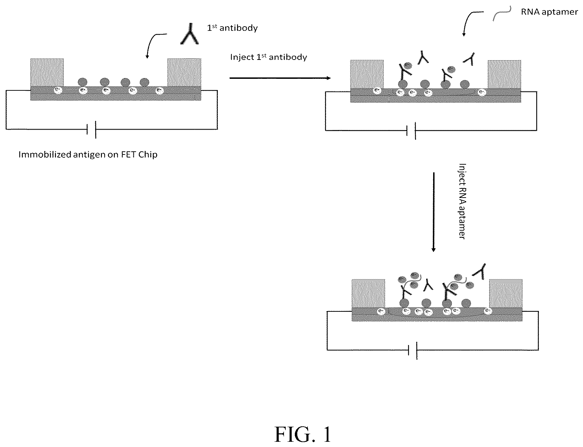

Referring to FIG. 1, first, an antigen as a recognizing molecule is immobilized on a surface of a FET chip, and a first antibody as a target molecule is injected. A capturing complex is formed with the antigen and the first antibody. In this embodiment, the capturing complex carries negative net charges. Furthermore, an RNA aptamer as a signal amplifier is injected and binds to the capturing complex. Because the RNA amptamer carries more negative net charges, the signal is amplified.

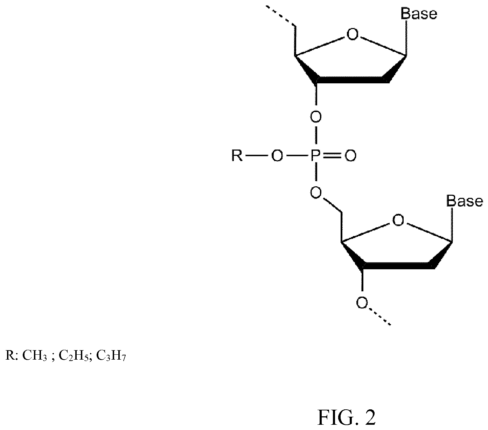

According to the invention, the recognizing molecule is a partially neutral single-stranded oligonucleotide comprising at least one electrically neutral nucleotide and at least one negatively charged nucleotide. The manner of rendering a nucleotide electrically neutral is not limited. In one embodiment of the invention, the electrically neutral nucleotide comprises a phosphate group substituted by an alkyl group. Preferably, the alkyl group is a C₁-C₆ alkyl group; more preferably, the alkyl group is a C₁-C₃ alkyl group. Examples of the C₁-C₃ alkyl group include but are not limited to methyl, ethyl and propyl. FIG. 2 shows one preferred embodiment of the electrically neutral nucleotide according to the invention. The negatively-charged oxygen atom in the phosphate group is changed to a neutral atom without charge. The way to substitute the phosphate group with the alkyl group can be applied according to common chemical reactions.

The negatively charged nucleotide according to the invention comprises a phosphate group with at least one negative charge. The unmodified nucleotide is preferably a naturally occurring nucleotide without modification or substitution. In one preferred embodiment of the invention, the negatively charged nucleotide comprises an unsubstituted phosphate group.

The partially neutral single-stranded oligonucleotide according to the invention is partially rendered electrically neutral. The sequence or length is not limited, and the sequence or length of the partially neutral single-stranded oligonucleotide can be designed according to a target molecule based on the disclosure of the invention.

The numbers of electrically neutral nucleotides and negatively charged nucleotides depend on the sequence of the partially neutral single-stranded oligonucleotide and the condition under the complex forming. The positions of the electrically neutral nucleotides and negatively charged nucleotides also depend on the sequence of the partially neutral single-stranded oligonucleotides and the condition under the complex formation. The numbers and positions of the electrically neutral nucleotides and negatively charged nucleotides can be designed according to the available information based on the disclosure of the invention. For example, the number and position of the electrically neutral nucleotides can be designed by molecular modeling calculation based on double stranded (ds) structural energy, and the melting temperature (Tm) of dsDNA/DNA or dsDNA/RNA can then be determined by reference to the structural energy.

In one preferred embodiment of the invention, the partially neutral single-stranded oligonucleotide comprises a plurality of the electrically neutral nucleotides, and at least one negatively charged nucleotide is positioned between two of the electrically neutral nucleotides; more preferably, at least two negatively charged nucleotides are positioned between two of the electrically neutral nucleotides.

By introducing the electrically neutral nucleotide, the melting temperature difference between perfect match double-stranded oligonucleotides and mismatched double-stranded oligonucleotides of the partially neutral single-stranded oligonucleotide according to the invention is higher compared with that of a conventional DNA probe. Without being restricted by theory, it is surmised that the electrostatic repulsion force between two strands is lowered by introducing the neutral oligonucleotide, and the melting temperature is raised thereby. By controlling the number and position of electrically neutral nucleotides, the melting temperature difference is adjusted to a desired point, providing a better working temperature or temperature range to differentiate the perfect and mismatched oligonucleotides, thereby improving capture specificity. Such design benefits the consistency of the melting temperature of different partially neutral single-stranded oligonucleotides integrated in one chip or array. The number of reactions to be detected can be raised dramatically with high specificity and more detection units can be incorporated into a single detection system. The design provides better microarray operation conditions.

In one preferred embodiment of the invention, the partially neutral single-stranded oligonucleotide comprises a first portion attached to the solid surface; the length of the first portion is about 50% of the total length of the partially neutral single-stranded oligonucleotide; and the first portion comprises at least one electrically neutral nucleotide and at least one negatively charged nucleotide; more preferably, the length of the first portion is about 40% of the total length of the partially neutral single-stranded oligonucleotide; still more preferably, the length of the first portion is about 30% of the total length of the partially neutral single-stranded oligonucleotide.

In one preferred embodiment of the invention, the partially single-stranded nucleotide further comprises a second portion adjacent to the first portion. The second portion is located in the distal end to the solid surface. The second portion comprises at least one electrically neutral nucleotide and at least one negatively charged nucleotide. The description of the electrically neutral nucleotide and the negatively charged nucleotide is the same as that of the first portion and is not repeated herein.

In one preferred embodiment of the invention, the method is performed in a buffer with an ionic strength lower than about 50 mM; more preferably lower than about 40 mM, 30 mM, 20 mM or 10 mM. Without being restricted by theory, it is surmised that by applying the partially neutral single-stranded oligonucleotide, the complex formed between the partially neutral single-stranded oligonucleotide with the target molecule can happen without the need to suppress the electrostatic repulsive forces between the partially charged semi-neutral single-stranded oligonucleotide and its target. The hybridization is then driven by the base pairing and the stacking force of each strand. Consequently, the duplex can be formed at a lower salt condition. With FET, the lower ion strength increases the detection length (the debye length) and, in turn, enhances the detection sensitivity.

In one embodiment of the invention, the improved hybridization specificity for forming the capturing complex can be seen mainly in two aspects of FET detection compared to a conventional detection. First, the melting temperature difference is higher. Second, the buffer has a lower salt condition, and the FET detection length (the debye length) is increased. Both of these differences result in improvement of detection sensitivity.

The present invention is also to provide a kit for detecting a target molecule, comprising:
a signal amplifier having affinity to a capturing complex comprising the target molecule, wherein the capturing complex has a net electrical charge; the signal amplifier has a like net electrical charge of the net electrical charge of the capturing complex; and
an electrical signal detecting element for detecting the electrical change occurring due to the binding of the capturing complex and the signal amplifier;
a recognizing molecule which is able to capture the target molecule with affinity to form the capturing complex, wherein the recognizing molecule comprises a partially neutral single-stranded oligonucleotide comprising at least one electrically neutral nucleotide and at least one negatively charged nucleotide; and
a solid surface, wherein the capturing complex is attached on the solid surface or the capturing complex is spaced apart from the solid surface by a distance of about 0 to about 10 nm.

Preferably, the kit according to the invention further comprises the recognizing molecule as mentioned above.

The following examples are provided.

### EXAMPLES

### Synthesis of a partially neutral single-stranded oligonucleotide as a recognizing molecule:

Deoxy cytidine (n-ac) p-methoxy phosphoramidite, thymidine p-methoxy phosphoramidite, deoxy guanosine (n-ibu) p-methoxy phosphoramidite, and deoxy adenosine (n-bz) p-methoxy phosphoramidite (all purchased from ChemGenes Corporation, USA) were used to synthesize an oligonucleotide according to a given sequence based on solid-phase phosphotriester synthesis or by Applied Biosystems 3900 High Throughput DNA Synthesizer (provided by Genomics® Biosci & Tech or Mission Biotech).

The synthesized oligonucleotide was reacted with weak alkaline in toluene at room temperature for 24 hours, and the sample was subjected to ion-exchange chromatography to adjust the pH value to 7. After the sample was concentrated and dried, the partially neutral single-stranded oligonucleotide was obtained.

### Synthesis of an RNA aptamer as a signal amplifier

The cDNA R18 RNA aptamer was synthesized by a polymerase chain reaction (PCR) and transcription reaction.

The PCR reaction solution contained: 2 µL of 10X PCR buffer, 0.5 µL of 10 mM dNTP, 0.5 µL of 10 µM cDNA Primer 1 (purchased from Integrated Device Technology (IDT), USA), 0.5 µL of 10 µM cDNA Primer 2 (purchased from Integrated Device Technology (IDT), USA), 0.5 µL of 10 µM cDNA R18 RNA aptamer template (purchased from Integrated Device Technology (IDT), USA), 0.5 µL of Taq DNA polymerase and 15.5 µL of DI-water. The PCR program was: one cycle of 95°C for 1 min, 50°C for 1 min, and 72°C for 1 min; 40 cycles of 95°C for 45 sec, 55°C for 45 sec, and 72°C for 45 sec; and one cycle of 72°C for 3 min. The PCR product was purified and stored at -20°C.

The purified PCR product was subjected to a transcription reaction. The reaction mixture contained: 25 µL of 2X buffer, 2.5 µL of 1 µg PCR product, 17.5 µL of DI-water, and 5 µL of Enzyme T7 Express (T7 RiboMAX™ Express Large Scale RNA Production System, Promega, USA). The reaction mixture was reacted at 37°C for 2 hours. The transcription product was purified.

### Recognizing molecule attachment:

A Si nanowire (SiNW) chip was washed with 40 mL of acetone twice, with 40 mL of ethanol twice, and with 40 mL of DI-water twice. The surface was dried with nitrogen air and introduced with oxygen plasma for 30 sec. A solution containing 80 µL of 25% (3-Aminopropyl)triethoxysilane (APTES) and 4 mL of ethanol was used to modify the surface for 30 min. The chip was again washed with 40 mL of ethanol twice and heated at 120°C for 10 min. The chip was immersed in glutaraldehyde (3 mL of 10 mM sodium phosphate buffer and 1 mL of glutaraldehyde) in liquid for 1 hour at room temperature. The chip was washed with sodium phosphate buffer twice.

Five hundred µL of 0.33% 6X Histidine-tag peptide (purchased from abcam, U.K.) was as a recognized molecule, and dropped on the chip surface. The coated chip was washed with 10 mM sodium phosphate buffer, and then immersed in 4 mM NaBH₃CN for 30 min, and 1% BSA blocking buffer for 1 h. Before nitrogen air drying, the chip was washed with Tris-HCl for 10 min twice, and then with DI-water.

### Capturing and signal amplifying

The chip was equipped with a microfluidic system. Five mL/hr of 1 mM Bis-tris propane was introduced into the channel, and a signal was monitored as a baseline.

The target molecule of 0.33% of anti-6X histidine-tag rabbit antibody was introduced into the channel at 5 mL/hr for 10 min and then incubated for 30 min. The channel was washed with 1 mM Bis-tris propane buffer for 10min, and a signal was monitored as a primary signal.

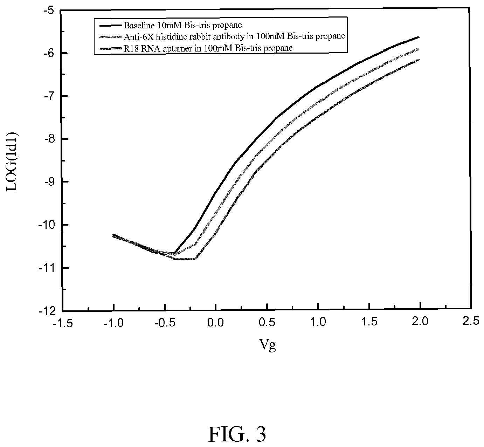

The signal amplifier of R18 RNA aptamer was introduced into the channel at 5 mL/hr for 10 min and then incubated for 30 min. The channel was washed with 1 mM Bis-tris propane buffer for 10 min, and a signal was monitored as an amplified signal. The result is shown in FIG. 3. The shift voltage is 122.6 mV (Δ*V*= *V*_{*d*1} - *V*_{*d*0}, *at I*_{*d*1} = -9).

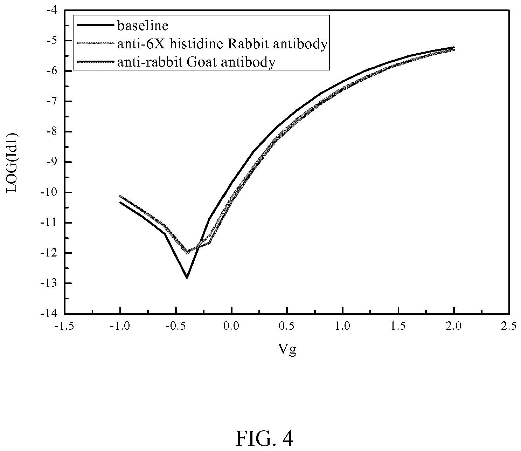

The anti-rabbit goat antibody as comparison was introduced into the channel at 5 mL/hr for 10 min and then incubated for 30 min. The channel was washed with 1 mM Bis-tris propane buffer for 10min, and a signal was monitored as a comparison signal. The result is shown in FIG. 4. The shift voltage is 20.9 mV (Δ*V*= *V*_{*d*1} - *V*_{*d*0}, *at I*_{*d*1} = -9).

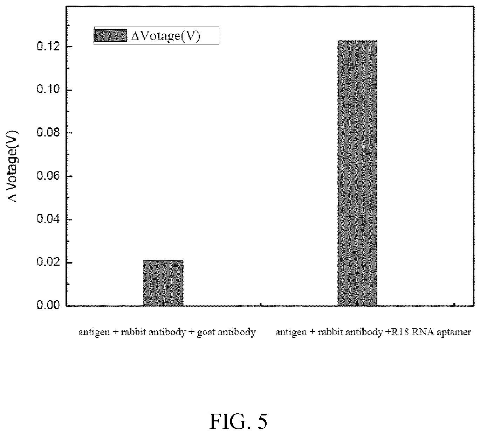

Referring to FIG. 5, the primary signal of the capturing complex was successfully and dramatically amplified by using the signal amplifier. Compared to the conventional secondary antibody (anti-rabbit Goat antibody), the signal amplifier according to the invention improves the sensitivity and sensing limit of detection.

## Claims

1. A method for detecting a target molecule, comprising forming a capturing complex comprising the target molecule; and binding the capturing complex with a signal amplifier, wherein the capturing complex has a net electrical charge; the signal amplifier has affinity to the capturing complex and has alike net electrical charge of the net electrical charge of the capturing complex, wherein the method further comprises forming the capturing complex with a recognizing molecule which is able to capture the target molecule with affinity, wherein the recognizing molecule comprises a partially neutral single-stranded oligonucleotide comprising at least one electrically neutral nucleotide and at least one negatively charged nucleotide, wherein the method further comprises monitoring an electrical change occurring due to the binding of the capturing complex and the signal amplifier, and wherein the capturing complex is attached on a solid surface or the capturing complex is spaced apart from the solid surface by a distance of about 0 to about 10 nm.

2. The method according to claim 1, wherein the solid surface is a transistor surface of a field-effect transistor (FET) or a metal surface of a surface plasmon resonance (SPR).

3. The method according to claim 1, wherein the material of the solid surface is polycrystalline silicon or single crystalline silicon.

4. The method according to claim 1, wherein the signal amplifier is an oligonucleotide molecule.

5. The method according to claim 1, wherein the signal amplifier is an oligonucleotide aptamer.

6. The method according to claim 3, wherein the solid surface is coupled with an electrical signal detecting element for detecting the electrical change occurring due to the binding of the capturing complex and the signal amplifier.

7. The method according to claim 6, wherein the electrical signal detecting element is a field-effect transistor or a surface plasmon resonance.

8. The method according to claim 1, wherein the electrically neutral nucleotide comprises a phosphate group substituted by a C₁-C₆ alkyl group.

9. The method according to claim 1, wherein the negatively charged nucleotide comprises an unsubstituted phosphate group.

10. The method according to claim 1, wherein the recognizing molecule is attached on a solid surface, and the partially neutral single-stranded oligonucleotide comprises a first portion attached to the solid surface; the length of the first portion is about 50% of the total length of the partially neutral single-stranded oligonucleotide; and the first portion comprises at least one electrically neutral nucleotide and at least one negatively charged nucleotide.

11. A kit for detecting a target molecule, comprising:
a signal amplifier having affinity to a capturing complex comprising the target molecule, wherein the capturing complex has a net electrical charge; the signal amplifier has alike net electrical charge of the net electrical charge of the capturing complex;
an electrical signal detecting element for detecting the electrical change occurring due to the binding of the capturing complex and the signal amplifier;
a recognizing molecule which is able to capture the target molecule with affinity to form the capturing complex, wherein the recognizing molecule comprises a partially neutral single-stranded oligonucleotide comprising at least one electrically neutral nucleotide and at least one negatively charged nucleotide; and
a solid surface, wherein the capturing complex is attached on the solid surface or the capturing complex is spaced apart from the solid surface by a distance of about 0 to about 10 nm.

12. The kit according to claim 11, wherein the solid surface is a transistor surface of a field-effect transistor (FET) or a metal surface of a surface plasmon resonance (SPR).

13. The kit according to claim 11, wherein the material of the solid surface is polycrystalline silicon or single crystalline silicon.

14. The kit according to claim 11, wherein the signal amplifier is an oligonucleotide molecule.

15. The kit according to claim 11, wherein the signal amplifier is an oligonucleotide aptamer.

16. The kit according to claim 13, wherein the solid surface is coupled with the electrical signal detecting element for detecting the electrical signal.

17. The kit according to claim 11, wherein the electrical signal detecting element is a field-effect transistor or a surface plasmon resonance.

18. The kit according to claim 11, wherein the electrically neutral nucleotide comprises a phosphate group substituted by a C₁-C₆ alkyl group.

19. The kit according to claim 11, wherein the negatively charged nucleotide comprises an unsubstituted phosphate group.

20. The kit according to claim 11, wherein the recognizing molecule is attached on a solid surface, and the partially neutral single-stranded oligonucleotide comprises a first portion attached to the solid surface; the length of the first portion is about 50% of the total length of the partially neutral single-stranded oligonucleotide; and the first portion comprises at least one electrically neutral nucleotide and at least one negatively charged nucleotide.

## Patentansprüche

1. Verfahren zum Detektieren eines Zielmoleküls, umfassend Bilden eines Einfangkomplexes, umfassend das Zielmolekül; und Binden des Einfangkomplexes mit einem Signalverstärker, wobei der Einfangkomplex eine elektrische Nettoladung aufweist; wobei der Signalverstärker Affinität zu dem Einfangkomplex aufweist und eine ähnliche elektrische Nettoladung wie die elektrische Nettoladung des Einfangkomplexes aufweist, wobei das Verfahren ferner Bilden des Einfangkomplexes mit einem Erkennungsmolekül, das zum Einfangen des Zielmoleküls mit Affinität in der Lage ist, umfasst, wobei das Erkennungsmolekül ein partiell neutrales einsträngiges Oligonukleotid, umfassend mindestens ein elektrisch neutrales Nukleotid und mindestens ein negativ geladenes Nukleotid, umfasst, wobei das Verfahren ferner Überwachen einer elektrischen Änderung, welche aufgrund der Bindung des Einfangkomplexes und des Signalverstärkers auftritt, umfasst, und wobei der Einfangkomplex auf einer festen Oberfläche gebunden ist oder der Einfangkomplex beabstandet von der festen Oberfläche durch einen Abstand von etwa 0 bis etwa 10 nm ist.

2. Verfahren gemäß Anspruch 1, wobei die feste Oberfläche eine Transistoroberfläche eines Feldeffekttransistors (FET) oder eine Metalloberfläche einer Oberflächenplasmonresonanz (SPR) ist.

3. Verfahren gemäß Anspruch 1, wobei das Material der festen Oberfläche polykristallines Silicium oder Einkristall-Silicium ist.

4. Verfahren gemäß Anspruch 1, wobei der Signalverstärker ein Oligonukleotid-Molekül ist.

5. Verfahren gemäß Anspruch 1, wobei der Signalverstärker ein Oligonukleotid-Aptamer ist.

6. Verfahren gemäß Anspruch 3, wobei die feste Oberfläche mit einem elektrisches Signal detektierenden Element zum Detektieren der elektrischen Änderung, welche aufgrund der Bindung des Einfangkomplexes und des Signalverstärkers auftritt, gekuppelt ist.

7. Verfahren gemäß Anspruch 6, wobei das elektrisches Signal detektierende Element ein Feldeffekttransistor oder eine Oberflächenplasmonresonanz ist.

8. Verfahren gemäß Anspruch 1, wobei das elektrisch neutrale Nukleotid eine Phosphatgruppe, substituiert mit einer C₁-C₆-Alkylgruppe, umfasst.

9. Verfahren gemäß Anspruch 1, wobei das negativ geladene Nukleotid eine nicht-substituierte Phosphatgruppe umfasst.

10. Verfahren gemäß Anspruch 1, wobei das Erkennungsmolekül auf einer festen Oberfläche gebunden ist und das partiell neutrale einsträngige Oligonukleotid einen ersten Teil, gebunden an die feste Oberfläche, umfasst; die Länge des ersten Teils etwa 50 % der Gesamtlänge des partiell neutralen einsträngigen Oligonukleotids beträgt; und der erste Teil mindestens ein elektrisch neutrales Nukleotid und mindestens ein negativ geladenes Nukleotid umfasst.

11. Kit zum Detektieren eines Zielmoleküls, umfassend:
einen Signalverstärker mit Affinität zu einem Einfangkomplex, umfassend das Zielmolekül, wobei der Einfangkomplex eine elektrische Nettoladung aufweist; der Signalverstärker eine ähnliche elektrische Nettoladung wie die elektrische Nettoladung des Einfangkomplexes aufweist;
ein elektrisches Signal detektierendes Element zum Detektieren der elektrischen Änderung, welche aufgrund der Bindung des Einfangkomplexes und des Signalverstärkers auftritt;
ein Erkennungsmolekül, welches zum Einfangen des Zielmoleküls mit Affinität in der Lage ist, wobei der Einfangkomplex gebildet wird, wobei das Erkennungsmolekül ein partiell neutrales einsträngiges Oligonukleotid, umfassend mindestens ein elektrisch neutrales Nukleotid und mindestens ein negativ geladenes Nukleotid, umfasst; und eine feste Oberfläche, wobei der Einfangkomplex auf der festen Oberfläche gebunden ist oder der Einfangkomplex beabstandet von der festen Oberfläche durch einen Abstand von etwa 0 bis etwa 10 nm ist.

12. Kit gemäß Anspruch 11, wobei die feste Oberfläche eine Transistoroberfläche eines Feldeffekttransistors (FET) oder eine Metalloberfläche einer Oberflächenplasmonresonanz (SPR) ist.

13. Kit gemäß Anspruch 11, wobei das Material der festen Oberfläche polykristallines Silicium oder Einkristall-Silicium ist.

14. Kit gemäß Anspruch 11, wobei der Signalverstärker ein Oligonukleotid-Molekül ist.

15. Kit gemäß Anspruch 11, wobei der Signalverstärker ein Oligonukleotid-Aptamer ist.

16. Kit gemäß Anspruch 13, wobei die feste Oberfläche mit dem elektrisches Signal detektierenden Element zum Detektieren des elektrischen Signals gekuppelt ist.

17. Kit gemäß Anspruch 11, wobei das elektrisches Signal detektierende Element ein Feldeffekttransistor oder eine Oberflächenplasmonresonanz ist.

18. Kit gemäß Anspruch 11, wobei das elektrisch neutrale Nukleotid eine Phosphatgruppe, substituiert mit einer C₁-C₆-Alkylgruppe, umfasst.

19. Kit gemäß Anspruch 11, wobei das negativ geladene Nukleotid eine nicht-substituierte Phosphatgruppe umfasst.

20. Kit gemäß Anspruch 11, wobei das Erkennungsmolekül auf einer festen Oberfläche gebunden ist und das partiell neutrale einsträngige Oligonukleotid einen ersten Teil, gebunden an die feste Oberfläche, umfasst; die Länge des ersten Teils etwa 50 % der Gesamtlänge des partiell neutralen einsträngigen Oligonukleotids beträgt; und der erste Teil mindestens ein elektrisch neutrales Nukleotid und mindestens ein negativ geladenes Nukleotid umfasst.

## Revendications

1. Procédé de détection d'une molécule cible, comprenant la formation d'un complexe de capture comprenant la molécule cible ; et la liaison du complexe de capture à un amplificateur de signal, dans lequel le complexe de capture présente une charge électrique nette ; l'amplificateur de signal présente une affinité pour le complexe de capture et présente la même charge électrique nette que la charge électrique nette du complexe de capture, le procédé comprenant en outre la formation du complexe de capture avec une molécule de reconnaissance capable de capturer la molécule cible par affinité, dans lequel la molécule de reconnaissance comprend un oligonucléotide simple brin partiellement neutre comprenant au moins un nucléotide électriquement neutre et au moins un nucléotide chargé négativement, le procédé comprenant en outre la surveillance d'une modification électrique résultant de la liaison du complexe de capture et de l'amplificateur de signal, et dans lequel le complexe de capture est fixé à une surface solide ou le complexe de capture est espacé de la surface solide d'une distance d'environ 0 à environ 10 nm.

2. Procédé selon la revendication 1, dans lequel la surface solide est une surface de transistor d'un transistor à effet de champ (FET) ou une surface métallique d'une résonance plasmonique de surface (SPR).

3. Procédé selon la revendication 1, dans lequel le matériau de la surface solide est du silicium polycristallin ou du silicium monocristallin.

4. Procédé selon la revendication 1, dans lequel l'amplificateur de signal est une molécule d'oligonucléotide.

5. Procédé selon la revendication 1, dans lequel l'amplificateur de signal est un aptamère d'oligonucléotide.

6. Procédé selon la revendication 3, dans lequel la surface solide est couplée à un élément de détection de signal électrique pour détecter la modification électrique résultant de la liaison du complexe de capture et de l'amplificateur de signal.

7. Procédé selon la revendication 6, dans lequel l'élément de détection de signal électrique est un transistor à effet de champ ou une résonance plasmonique de surface.

8. Procédé selon la revendication 1, dans lequel le nucléotide électriquement neutre comprend un groupe phosphate substitué par un groupe alkyle en C₁ à C₆.

9. Procédé selon la revendication 1, dans lequel le nucléotide chargé négativement comprend un groupe phosphate non substitué.

10. Procédé selon la revendication 1, dans lequel la molécule de reconnaissance est fixée à une surface solide et l'oligonucléotide simple brin partiellement neutre comprend une première partie fixée à la surface solide ; la longueur de la première partie est d'environ 50 % de la longueur totale de l'oligonucléotide simple brin partiellement neutre ; et la première partie comprend au moins un nucléotide électriquement neutre et au moins un nucléotide chargé négativement.

11. Kit de détection d'une molécule cible, comprenant :
un amplificateur de signal présentant une affinité pour un complexe de capture comprenant la molécule cible, dans lequel le complexe de capture présente une charge électrique nette ; l'amplificateur de signal présente la même charge électrique nette que la charge électrique nette du complexe de capture ;
un élément de détection de signal électrique pour détecter la modification électrique résultant de la liaison du complexe de capture et de l'amplificateur de signal ;
une molécule de reconnaissance capable de capturer la molécule cible par affinité pour former le complexe de capture, dans lequel la molécule de reconnaissance comprend un oligonucléotide simple brin partiellement neutre comprenant au moins un nucléotide électriquement neutre et au moins un nucléotide chargé négativement ; et
une surface solide, dans lequel le complexe de capture est fixé à la surface solide ou le complexe de capture est espacé de la surface solide d'une distance d'environ 0 à environ 10 nm.

12. Kit selon la revendication 11, dans lequel la surface solide est une surface de transistor d'un transistor à effet de champ (FET) ou une surface métallique d'une résonance plasmonique de surface (SPR).

13. Kit selon la revendication 11, dans lequel le matériau de la surface solide est du silicium polycristallin ou du silicium monocristallin.

14. Kit selon la revendication 11, dans lequel l'amplificateur de signal est une molécule d'oligonucléotide.

15. Kit selon la revendication 11, dans lequel l'amplificateur de signal est un aptamère d'oligonucléotide.

16. Kit selon la revendication 13, dans lequel la surface solide est couplée à un élément de détection de signal électrique pour détecter le signal électrique.

17. Kit selon la revendication 11, dans lequel l'élément de détection de signal électrique est un transistor à effet de champ ou une résonance plasmonique de surface.

18. Kit selon la revendication 11, dans lequel le nucléotide électriquement neutre comprend un groupe phosphate substitué par un groupe alkyle en C₁ à C₆.

19. Kit selon la revendication 11, dans lequel le nucléotide chargé négativement comprend un groupe phosphate non substitué.

20. Kit selon la revendication 11, dans lequel la molécule de reconnaissance est fixée à une surface solide, et l'oligonucléotide simple brin partiellement neutre comprend une première partie fixée à la surface solide; la longueur de la première partie est d'environ 50 % de la longueur totale de l'oligonucléotide simple brin partiellement neutre ; et la première partie comprend au moins un nucléotide électriquement neutre et au moins un nucléotide chargé négativement.
